# EUROPÄISCHE PATENTSCHRIFT

(11) **EP 1 357 849 B1**
(45) Veröffentlichungstag und Bekanntmachung des Hinweises auf die Patenterteilung: **07.10.2009**
(21) Anmeldenummer: 02708293.2
(22) Anmeldetag: 17.01.2002
(51) Int. Cl.: A61B 18/24, A61B 18/04

(54) **VORRICHTUNG ZUM APPLIZIEREN VON LICHT AUF EINE GEFÄSSWAND**
DEVICE FOR APPLYING LIGHT ON THE WALL OF A VESSEL
DISPOSITIF POUR L'APPLICATION D'UNE LUMIERE SUR UNE PAROI D'UN RECIPIENT

(30) Priorität: 19.01.2001 DE 10102477
(43) Veröffentlichungstag der Anmeldung: 05.11.2003
(73) Patentinhaber: Storz-Endoskop GmbH, 8200 Schaffhausen (CH)
(72) Erfinder: FRENZ, Martin, CH-3110 Münsingen (CH); OTT, Beat, CH-3800 Matten (CH)
(74) Vertreter: Heuckeroth, Volker
(86) Internationale Anmeldenummer: PCT/EP2002/000419
(87) Internationale Veröffentlichungsnummer: WO 2002/056785

(56) Entgegenhaltungen:
- EP-A- 0 821 916
- US-A- 5 380 317

## Beschreibung

Die Erfindung betrifft eine Vorrichtung zum Applizieren von Licht auf eine zu behandelnde Stelle einer Wand eines Gefäßes im menschlichen oder tierischen Körper, insbesondere zum Laserschweißen zweier Gefäße aneinander, mit einem lichtzuführenden Instrument, das von einer extrakorporalen Lichtquelle erzeugtes Licht zu der Stelle führt und auf diese abstrahlt, gemäß dem Oberbegriff des Anspruchs 1.

Eine solche Vorrichtung ist aus EP-A-0 761 257 bekannt.

Ein allgemeiner Anwendungsfall einer solchen Vorrichtung besteht in der Applikation von Licht auf eine zu behandelnde Stelle einer Wand eines Gefäßes, um einen pathologischen Zustand, bspw. eine Gewebeveränderung oder einen Bruch der Gefäßwand an dieser Stelle zu heilen.

Die aus dem o.g. Dokument EP-A-0 761 257 bekannte Vorrichtung zum Applizieren von Licht auf eine zu behandelnde Stelle einer Wand ist in Form einer Lasersonde ausgebildet, mit der Laserlicht gleichförmig auf eine zu behandelnde Stelle eingestrahlt werden kann. Die Lasersonde ist mit einem transparenten Lichtdiffusor zum Abstrahlen von Laserlicht versehen, das von einer Lichtleitfaser axial zugeführt und von dem Lichtdiffusor radial bzgl. der Längsrichtung der Lichtleitfaser abgestrahlt wird. Am Umfang des Lichtdiffusors ist eine Mehrzahl von reflektierenden Nuten vorgesehen, die sich senkrecht zur Längsrichtung des Lichtdiffusors und zur Längsrichtung der Lichtleitfaser erstrecken. Diese Vorrichtung zum Applizieren von Licht wird im Rahmen der fotodynamischen Therapie verwendet.

Ein spezieller Anwendungsfall der eingangs genannten Vorrichtung, auf den sich die nachfolgende Beschreibung bezieht, ohne hierauf beschränkt zu sein, ist die Anwendung einer solchen Vorrichtung zum Laserschweißen zweier Gefäße aneinander, um diese Ende an Ende oder auch Ende an Seite, so daß eine T-förmige Verbindung hergestellt wird, miteinander zu verbinden. Unter Gefäßen sind Blutgefäße wie Venen oder Arterien, oder auch im weiteren Sinne allgemein Hohlorgane zu verstehen. Eine solche Verbindung zweier Gefäße miteinander ist bspw. erforderlich, wenn ein Gefäß auf grund Verletzung oder aufgrund einer operativen Resektion zertrennt wurde, oder um zwei Gefäße ineinander mündend zu verbinden, um einen neuen Gefäßgang zu schaffen oder einen ursprünglichen wieder herzustellen.

Bei herkömmlichen Verfahren zum Verbinden zweier Gefäßstümpfe Ende an Ende miteinander, das auch als Anastomose bezeichnet wird, wurden die beiden Gefäßstümpfe durch herkömmliche Nahtverfahren miteinander verbunden. Derartige Nahtverfahren sind jedoch zeitaufwendige chirurgische Verfahren, die seitens des Chirurgen Erfahrung und Geschick erfordern. Des weiteren besteht beim Zusammennähen zweier Gefäßstümpfe die Gefahr, daß sich an der Nahtstelle ein Aneurisma, d.h. eine Gefäßwandschwächung mit einhergehender Aussackung der Gefäßwand entwickelt, oder daß Fremdkörperreaktionen aufgrund des Nahtmaterials auftreten. Außerdem lassen sich Nahtverbindungen bei Gefäßen oft nur in offenen, d.h. invasiven Eingriffen herstellen.

Durch die Entwicklung der Laserchirurgie wurde es alsdann möglich, Nahtverbindungen durch Laserschweißen zu ersetzen, wie bspw. in US-A-5 827 265 offenbart. Es hat sich herausgestellt, daß sich mit Laserlicht geeigneter Wellenlänge und geeigneter Leistung auch Gewebe von Gefäßen "verschmelzen" läßt. Es wurden bereits erfolgreiche Versuche in vitro zum Verschweißen zweier Gefäßstümpfe Ende an Ende durchgeführt. Der Vorteil der Laserbehandlung gegenüber den herkömmlichen Nahttechniken besteht in einem geringeren Zeitaufwand und in dem Vermeiden von Fremdkörperreaktionen durch Nahtmaterial. Außerdem können Laserschweißtechniken auch endoskopisch durch kleine Inzisionen, d.h. minimal-invasiv, durchgeführt werden.

Obwohl die Mechanismen des Laserschweißprozesses bei Gefäßgewebe noch nicht vollständig verstanden werden, wird vermutet, daß das Laserlicht, das das Gewebe lokal erwärmt, zu einer Koagulation von Proteinen und damit zu einer anastomotischen Verbindung von Gefäßstümpfen führt. Jedoch zeigen mit Laserlicht verschweißte Gefäße häufig eine ungenügende Stabilität der Verbindung, die unter der Beanspruchung des in dem Gefäß pulsierenden Blutes zu Undichtigkeiten oder gar einem vollständigen Aufbrechen der Verbindung führen können.

Es wurde daher vorgeschlagen, auf die Verbindungsstelle vor dem Schweißen ein biologisches Lot wie Fibrin oder Albumin in flüssiger oder fester Form aufzutragen, die in das zu verschweißende Gewebe eindringen und beim Verschweißen als Klebstoff wirken. Zusätzlich werden diese mit einem Chromophor als Absorber gemischt, dessen Absorptionsmaximum bei der Wellenlänge des verwendeten Laserlichts liegt, um das Laserlicht optimal in das Lot einzukoppeln und die Laserenergie dort freizusetzen.

Ein Problem stellt allerdings die Applikation des Lichtes in vivo auf die zu behandelnde Stelle an der Wand des Gefäßes dar. Bei einer Applikation des Lichtes von der Außenseite der Wand des Gefäßes her, erweist es sich als schwierig, eine solche Stelle an der Wand des Gefäßes zu behandeln, die auf der der Inzision abgewandten Seite des Gefäßes liegt. Insbesondere bei einer lasergestützten Anastomose, bei der zwei getrennte Gefäße aneinandergeschweißt werden sollen, müßte das lichtzuführende Instrument ein lichtaustrittseitiges Ende aufweisen, das über einen vollen Umfang um die Außenseite der Wand des Gefäßes herum geführt werden kann. Dies könnte bspw. durch eine Zange erfolgen, dessen distales Ende einen spreizbaren zylindrischen Greifer aufweist, mit dem das Gefäß zangenartig umgriffen wird, und bei dem auf der Innenseite der Greifer das Licht umfänglich austritt. Dabei besteht jedoch die Gefahr, daß beim Schweißen der Gefäße diese kollabieren, mit dem Ergebnis, daß nach Herstellen der Schweißverbindung das Gefäß nicht mehr für den Durchgang von Blut offen ist. Außerdem erfordert das Einführen einer solchen Vorrichtung in den Körper möglicherweise einen offenen invasiven Eingriff.

Die aus dem Dokument US-A-5 827 265 bekannte Vorrichtung zum Laserschweißen zweier Gefäße aneinander weist einen Katheter auf, der an einem Ende mit einer optischen Faser verbunden ist, an der zumindest ein aufblasbarer Ballon angebracht ist. Der Ballon ist mit einem Collagenband umwickelt, das sich beim Aufblasen des Ballons an die Gefäßinnenwand legt. Das Collagenband weist eine dünne Schicht eines lichtabsorbierenden Stoffes auf. Laserlicht wird von der optischen Faser emittiert und durch den Ballon hindurch gestreut, um eine gleichmäßige Beleuchtung des Collagenbandes zu gewährleisten.

Ferner ist aus US-A-5 649 924 eine Vorrichtung zum Applizieren von Licht auf eine zu behandelnde Stelle einer Körperhöhle, insbesondere der Gebärmutter, bekannt. Bei dieser Vorrichtung wird über einen Lichtleiter Laserlicht zugeführt, an dessen distalem Ende ein Reflektor angeordnet ist, der das von dem Lichtleiter emittierte Licht radial abstrahlt, und zwar in einem umfänglich begrenzten Bereich. Der Reflektor ist teilweise als Lichtabsorber ausgebildet, um durch die Lichtabsorption Wärme im distalen Ende des Katheters zu erzeugen. Auf die zu behandelnde Stelle wird somit Laserlichtenergie als auch Wärmeenergie gleichzeitig appliziert.

Der Erfindung liegt die Aufgabe zugrunde, eine Vorrichtung der eingangs genannten Art zu schaffen, mit der auf minimalinvasivem Wege das Licht auf die zu behandelnde Stelle der Wand des Gefäßes appliziert werden kann, und mit der mit geringem Zeitaufwand insbesondere eine Laserschweißung zweier Gefäße durchgeführt werden kann.

Erfindungsgemäß wird diese Aufgabe hinsichtlich der eingangs genannten Vorrichtung durch die kennzeichnenden Merkmale des Anspruchs 1 gelöst.

Die erfindungsgemäße Vorrichtung ermöglicht es, das zu applizierende Licht von der Innenseite des Wand des Gefäßes auf die zu behandelnde Stelle zu applizieren. Der längliche Lichtleiter läßt sich wie eine Kanüle oder ein Katheter an einer Stelle abseits der zu behandelnden Stelle in das Lumen des Gefäßes einführen und darin in Längsrichtung des Gefäßes an die zu behandelnde Stelle verschieben. Der längliche Lichtleiter führt somit das von der extrakorporalen Lichtquelle erzeugte Licht zunächst axial längs des Gefäßes zu der zu behandelnden Stelle. Im Bereich der zu behandelnden Stelle richten die erfindungsgemäß vorgesehenen lichtablenkenden Mittel das durch den Lichtleiter axial zugeführte Licht dann im wesentlichen radial auf die zu behandelnde Stelle. Damit der Lichtleiter in das Lumen des Gefäßes einführbar ist, weist der Lichtleiter einen entsprechenden Durchmesser auf, der kleiner ist als der Innendurchmesser des Gefäßes. Es ergibt sich, daß sich mit der erfindungsgemäßen Vorrichtung die Lichtapplikation auf die zu behandelnde Stelle der Wand des Gefäßes minimal-invasiv durchführen läßt. Bei einer Behandlung einer Gefäßwandstelle bspw. in der Bauchaorta kann der Lichtleiter bspw. im Bereich der Hüfte in die Hüftarterie eingeführt und an die gewünschte Stelle vorgeschoben werden. Der weitere Vorteil der erfindungsgemäßen Vorrichtung besteht darin, daß der in das Gefäß eingeführte Lichtleiter ein Kollabieren des Gefäßes im Bereich der Behandlungsstelle vermeidet, sondern den Durchgang des Gefäßes offen hält. Die erfindungsgemäße Vorrichtung eignet sich insbesondere für die lasergestützte Anastomose zum Verbinden zweier Gefäße Ende an Ende oder Ende an Seite aneinander, um zwei Beispiele zu nennen.

Der Lichtleiter ist an einem distalen Ende gerade, so daß das Licht aus dem Lichtleiter im wesentlichen axial austritt, wobei die lichtablenkenden Mittel das aus dem Lichtleiter austretende Licht in im wesentlichen radialer Richtung zur behandelnden Stelle hinlenken.

Die am distalen Ende des Lichtleiters gerade Ausgestaltung hat über der zuvor beschriebenen am distalen Ende abgebogenen Ausgestaltung den Vorteil, daß der Lichtleiter insgesamt mit größerem Querschnitt ausgebildet werden kann, wodurch durch den Lichtleiter mehr Lichtenergie zu der zu behandelnden Stelle pro Zeiteinheit geführt werden kann.

Die lichtablenkenden Mittel sind so ausgebildet, daß sie das aus dem Lichtleiter austretende Licht ohne Drehen des Lichtleiters um seine Längsachse über einen vollen Umfang gleichmäßig verteilt, insbesondere ringförmig zur zu behandelnden Stelle an der Wand des Gefäßes hin lenken.

Diese Maßnahme ist speziell dann von Vorteil, wenn der gesamte Umfang der Wand des Gefäßes mit Licht behandelt werden soll, wie dies bei der Laserschweißung zweier Gefäße aneinander der Fall ist. Das Laserschweißen kann somit mit noch geringerem Zeitaufwand durchgeführt werden, weil der gesamte Umfang der Wand des Gefäßes gleichzeitig mit Licht bestrahlt wird. Gleichzeitig wird ein über den gesamten Umfang des Gefäßes gesehen gleichmäßiges Ergebnis der Verschweißung der Gefäße aneinander erreicht, was mit einem von Hand zu drehenden Lichtleiter nur schwer zu kontrollieren ist.

Die lichtablenkenden Mittel sind als Reflektor ausgebildet, dessen reflektierende Fläche zur Längsrichtung des Lichtleiters mit einem Winkel im Bereich zwischen 40° und 50°, vorzugsweise etwa 45°, geneigt ist.

Die Ausgestaltung der lichtablenkenden Mittel als Reflektor haben den Vorteil, daß Lichtverluste aufgrund von Absorption oder Streuung bei der Richtungsablenkung des Lichts minimiert werden können. Die reflektierende Fläche kann bspw. aus Silber bestehen.

Dabei ist es bevorzugt, wenn die reflektierende Fläche als Konusfläche, insbesondere spitzkegelförmig ausgebildet ist.

Diese Maßnahme stellt eine konstruktiv einfache Ausgestaltung eines Reflektors dar, der es ermöglicht, das aus dem Lichtleiter austretende Licht ohne' Drehen des Lichtleiters um seine Längsachse über einen vollen Umfang gleichmäßig verteilt ringförmig zur zu behandelnden Stelle an der Wand des Gefäßes hin zu lenken. Eine spitzkegelförmige Ausgestaltung hat des weiteren den Vorteil, daß an der Spitze des Kegels so gut wie keine Lichtverluste aufgrund von Streuung auftreten.

In einer weiteren bevorzugten Ausgestaltung, ist die reflektierende Fläche gerade, konkav gekrümmt oder konvex gekrümmt.

Die Wahl der Geometrie der reflektierenden Oberfläche kann zur Optimierung des auf die zu behandelnde Stelle an der Wand des Gefäßes gerichteten Lichtstrahls verwendet werden. Mit einer konkav geformten reflektierenden Fläche kann beispielsweise eine Fokussierung des Lichts erreicht werden, mit einer konvex gekrümmten reflektierenden Fläche eine Streuung bzw. Divergenz des Lichts, um einen breiteren Lichtfleck bzw. Lichtring an der Gefäßwand zu erhalten.

In einer weiteren bevorzugten Ausgestaltung sind das distale Ende des Lichtleiters und die lichtablenkenden Mittel relativ zueinander unbeweglich gehalten.

Diese Maßnahme hat den Vorteil, daß sich das distale Ende des Lichtleiters während eines Einsatzes des lichtzuführenden Instruments und die lichtablenkenden Mittel nicht relativ zueinander verschieben, verdrehen oder verkippen, so daß gewährleistet ist, daß das zu applizierende Licht stets genau auf die zu behandelnde Stelle an der Wand des Gefäßes gerichtet bleibt.

In einer weiteren bevorzugten Ausgestaltung ist das lichtzuführende Instrument als Katheter ausgebildet.

Hierbei ist von Vorteil, daß der das lichtzuführende Instrument bedienende Chirurg, üblicherweise ein Gefäßchirurg, bereits an die Handhabung von Kathetern gewöhnt ist und sich nicht auf das neue Instrument hinsichtlich seiner Handhabung neu einstellen muß.

Dabei ist es bevorzugt, wenn der Katheter einen den Lichtleiter einhüllenden Mantel aufweist, der zumindest im Bereich der lichtablenkenden Mittel für das applizierende Licht transparent ist.

Hierbei ist von Vorteil, daß das zu applizierende Licht ohne Schwächung durch den Mantel, der die Hülle des Katheters bildet, hindurchtreten kann und mit der im wesentlichen an der Lichtquelle eingestellten gewünschten Leistung auf die zu behandelnde Stelle eingestrahlt werden kann.

Weiterhin ist es bevorzugt, wenn ein distales Ende des Lichtleiters und die lichtablenkenden Mittel in einem beide verbindenden, für das zu applizierende Licht transparenten Röhrchen angeordnet sind.

Diese Maßnahme stellt eine konstruktiv einfache Möglichkeit dar, die lichtablenkenden Mittel in Form des vorzugsweise konusförmigen Reflektors fest mit dem distalen Ende des Lichtleiters unbeweglich zueinander zu verbinden. Das transparente Röhrchen kann bspw. aus Plexiglas gefertigt sein.

In einer weiteren bevorzugten Ausgestaltung weist das lichtzuführende Instrument Haltemittel zum Fixieren des Gefäßes, insbesondere von Gefäßen aneinander, während der Lichtapplikation auf.

Insbesondere beim Verbinden zweier Gefäße durch Laserschweißen miteinander ist es erforderlich, die beiden Gefäße während der Lichtapplikation aneinander zu fixieren, bis eine ausreichende Verschmelzung stattgefunden hat. Durch die zuvor genannten am Instrument vorgesehenen Haltemittel werden zum Fixieren somit keine Nähte benötigt, die sich, wie eingangs erwähnt, als nachteilig erwiesen haben. Die Haltemittel können bspw. in einem das Instrument umgebenden Gefäßstent bestehen, wie er in der Gefäßchirurgie häufig verwendet wird.

In einer besonders bevorzugten Ausgestaltung weisen die Haltemittel jedoch einen Ballon auf, der sich axial über das distale Ende des Lichtleiters und über die lichtablenkenden Mittel hinweg erstreckt und den Lichtleiter und die lichtablenkenden Mittel umgibt.

Das erfindungsgemäße Instrument ist somit vorzugsweise als Ballonkatheter ausgebildet, wobei der Ballon dazu dient, die beiden Gefäße für den Schweißvorgang aneinander zu fixieren. Außerdem ermöglicht der Ballon eine sehr genau Ausrichtung der beiden Gefäße zueinander, beispielsweise bei einer Ende-an-Ende-Verbindung, ohne Achsenversatz, wodurch eine sehr saubere Schweißnaht zwischen den Gefäßen hergestellt werden kann. Der Durchmesser des Ballons ist vorzugsweise so gewählt, daß er geringfügig größer ist als der Innendurchmesser des Lumens der Gefäße, wodurch die Gefäße fest und unbeweglich auf dem Ballon fixiert werden können.

In einer weiteren bevorzugten Ausgestaltung weist der Ballon eine lang erstreckte Gerade oder T-förmige Geometrie auf.

Mit einer lang erstreckten geraden Form des Ballons lassen sich zwei Gefäße für eine Ende-an-Ende-Verbindung in genauer axialer Ausrichtung zueinander aneinander für den Schweißvorgang fixieren, während sich durch eine T-förmige Geometrie des Ballons zwei Gefäße Ende an Seite gut aneinander fixieren lassen.

Dabei ist es insbesondere bevorzugt, wenn der Ballon dilatierbar ist.

Dies hat zum einen den Vorteil, daß der Ballon an verschiedene Durchmesser von Lumina von Gefäßen durch verschieden starkes Dilatieren, d.h. Aufweiten mittels bspw. eines Fluides, anpaßbar ist, zum anderen hat diese Maßnahme den Vorteil, daß für den Vorgang des Einführens des Katheters in das Gefäß und zum Herausziehen aus dem Gefäß der Ballon durch Ablassen des Fluids im Durchmesser verkleinerbar ist, wodurch sich das Instrument sehr leicht in dem Gefäß verschieben und insbesondere durch eine sehr kleine Inzision in das Gefäß einführen und wieder entnehmen läßt. Ein mit einem Fluid, bspw. Wasser, füllbarer Ballon hat darüber hinaus den Vorteil einer Kühlung.

Weiterhin ist es bevorzugt, wenn eine Außenseite des Ballons aus einem nicht an biologischem Gewebe anhaftenden Material besteht.

Hierdurch wird vorteilhafterweise vermieden, daß beim Behandeln der Gefäßwand mit Licht, insbesondere beim Verschweißen zweier Gefäßstümpfe, das Gefäßgewebe an dem Ballon "anbackt". Nach Beendigung des Behandlungsvorganges läßt sich der Ballon somit problemlos von der Gefäßwand lösen.

In einer weiteren bevorzugten Ausgestaltung ist der Katheter flexibel.

Hierbei ist von Vorteil, daß sich das lichtzuführende Instrument auch in nicht geraden Gefäßbögen einführen und darin axial verschieben läßt.

In einer weiteren bevorzugten Ausgestaltung weist der Lichtleiter eine einzelne Lichtleitfaser auf.

Mit einer einzelnen Lichtleitfaser kann das erfindungsgemäße lichtzuführende Instrument, insbesondere in Ausgestaltung als Katheter, sehr dünn im Durchmesser gebaut werden und kann eine entsprechende Flexibilität besitzen. Die Lichtleitfaser ist vorzugsweise eine für Laseranwendungen übliche Glasfaser. Die Verwendung einer einzelnen Lichtleitfaser als Lichtleiter hat darüber hinaus den Vorteil, daß die Abstrahlung des Lichts aus dem Lichtleiter auf einen sehr kleinen Spot konzentriert werden und somit sehr präzise kontrolliert werden kann.

In einer weiteren bevorzugten Ausgestaltung erzeugt die Lichtquelle Licht, dessen Wellenlänge in einem Wellenlängenbereich zwischen 780 nm und 2100 nm liegt und vorzugsweise 808 nm oder 2010 nm beträgt.

Von den Erfindern wurden Versuche zum Aneinanderschweißen von Gefäßstümpfen durchgeführt, die bei den genannten Wellenlängen und den genannten Leistungen des Laserlichts sich hinsichtlich der erforderlichen Schweißdauer, der Zugfestigkeit, der Dichtigkeit, Bruchfestigkeit und der Gewebeschädigung als besonders vorteilhaft für eine Anastomose von Gefäßen erwiesen haben, wobei in Verbindung mit einem Gewebeklebstoff, bspw. Fibrin oder Albumin, besonders gute Ergebnisse erzielt werden.

Weitere Vorteile und Merkmale ergeben sich aus der nachfolgenden Beschreibung und der beigefügten Zeichnung.

Es versteht sich, daß die vorstehend genannten und nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden mit Bezug auf diese hiernach näher beschrieben. Es zeigen:
- Fig. 1: Eine schematische ausschnittsweise Darstellung einer Vorrichtung zum Applizieren von Licht auf eine zu behandelnde Stelle einer Wand eines Gefäßes im menschlichen oder tierischen Körper in Seitenan- sicht, teilweise im Längsschnitt;
- Fig. 2: einen vergrößerten Ausschnitt der Vorrichtung in Fig. 1;
- Figuren 3a) bis 3c): eine schematische Darstellung der Anwendung der Vor- richtung in Figuren 1 und 2 in drei Teilbildern zum Verschweißen zweier Gefäße aneinander; und
- Fig. 4: eine schematische ausschnittsweise Darstellung eines gegenüber Fig. 1 abgewandelten Ausführungsbeispiels.

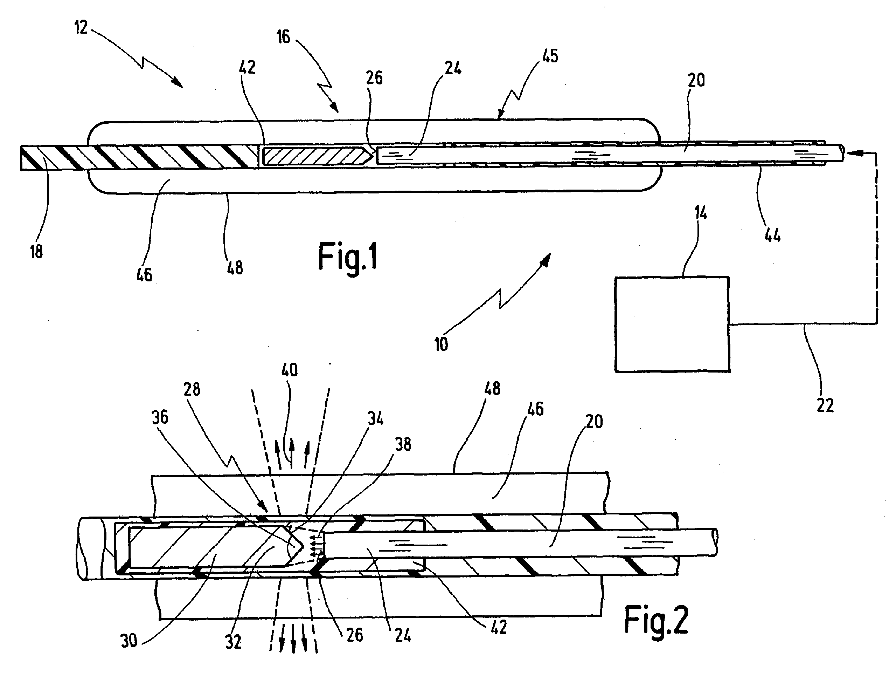

In Fig. 1 ist eine mit dem allgemeinen Bezugszeichen 10 dargestellte Vorrichtung zum Applizieren von Licht auf eine zu behandelnde Stelle einer Wand eines Gefäßes im menschlichen oder tierischen Körper, insbesondere zum Laserschweißen zweier Gefäße aneinander, schematisch dargestellt.

Die Vorrichtung 10 weist ein lichtzuführendes Instrument 12 auf, das von einer extrakorporalen Lichtquelle 14 erzeugtes Licht zu der zu behandelnden Stelle führt. Die Darstellung des Instruments 12 und der Lichtquelle 14 ist nicht maßstabsgetreu, vielmehr ist das Instrument 12 im Verhältnis zur Lichtquelle 14 stark vergrößert dargestellt.

Das lichtzuführende Instrument 12 ist als Katheter 16 ausgebildet. Ein distales Ende 18 des Katheters 16 bildet die Katheterspitze. Der Katheter 16 ist so ausgebildet, daß er in das Lumen eines Gefäßes, bspw. eine Vene oder Arterie, eingeführt und darin in Längsrichtung des Gefäßes verschoben werden kann. Das distale Ende 18 des Katheters 16 kann auch mit einer Spitze ausgebildet sein, um durch die Gefäßwand in das Lumen des Gefäßes gestochen werden zu können.

Das Instrument 12 weist einen länglichen Lichtleiter 20 auf, der als einzelne Lichtfaser ausgebildet ist. Der Lichtleiter 20 kann jedoch auch aus einem Bündel von Lichtleitfasern ausgebildet sein. Die den Lichtleiter 20 bildende Lichtleitfaser ist eine Glasfaser.

Das in der Lichtquelle 14 extrakorporal erzeugte Licht, bspw. Laserlicht, wird bspw. über ein schematisch dargestelltes Lichtleitkabel 22, das am nicht näher dargestellten proximalen Ende des Katheters 16 mit diesem verbunden ist, in den Lichtleiter 20 extrakorporal eingekoppelt.

Ein distales Ende 24 des Lichtleiters 20 ist gerade ausgebildet. Durch den Lichtleiter 20 von proximal nach distal geführtes Licht tritt daher aus einer Stirnfläche 26 des Lichtleiters im wesentlichen axial aus.

Das lichtzuführende Instrument weist weiterhin lichtablenkende Mittel 28 auf, die das durch den Lichtleiter zugeführte Licht im wesentlichen radial und somit quer zur Längsrichtung des Lichtleiters 20 ablenken.

Die lichtablenkenden Mittel 28 sind als Reflektor 30 ausgebildet, der durch ein zylinderförmiges Element gebildet wird, dessen dem distalen Ende 24 des Lichtleiters 20 zugewandtes proximales Ende 32 eine Reflektorfläche 34 aufweist. Die reflektierende Fläche 34 ist als Konusfläche, im gezeigten Ausführungsbeispiel als Mantelfläche eines Spitzkegels, ausgebildet.

Während die als Mantelfläche eines Spitzkegels ausgebildete reflektierende Fläche 34 im gezeigten Ausführungsbeispiel gerade ist, kann zur Fokussierung des reflektierenden Lichts die Kegelfläche auch konkav oder zur Streuung des Lichts konvex gekrümmt sein.

Während der Körper des Reflektors 30 aus Kunststoff ausgebildet sein kann, weist die Reflektorfläche 34 ein Material mit einem hohen Reflexionskoeffizienten, bspw. Silber, auf.

Die Reflektorfläche 34 weist einen Öffnungswinkel 36 von etwa 90° auf, so daß die reflektierende Fläche 34 zur Längsrichtung des Lichtleiters 20 unter einem Winkel von etwa 45° geneigt ist.

Aufgrund der Ausgestaltung der reflektierten Fläche 34 als Konusfläche wird das aus der Stirnfläche 26 des Lichtleiters 20 im wesentlichen axial austretende Licht (Pfeile 38) über einen vollen Umfang von 360°, das heißt allseitig, gleichmäßig verteilt im wesentlichen radial ringförmig abgelenkt.

Die Abmessungen des Lichtleiters 20 und der reflektierenden Fläche 34 des Reflektors 30 sind so bemessen, daß das gemäß den Pfeilen 40 reflektierte Licht im in das Gefäß eingesetzten Zustand des Katheters 16 ringförmig auf die Wand des Gefäßes auftrifft, wobei der Ringstreifen eine Breite von etwa 1 bis 5 mm aufweist.

Die lichtablenkenden Mittel 28 in Form des Reflektors 30 und das distale Ende 24 des Lichtleiters 20 sind relativ zueinander unbeweglich gehalten. Dazu ist der Reflektor 30 und das distale Ende 24 des Lichtleiters 20 in einem beide verbindenden und für das zu applizierende Licht transparenten Röhrchen 42 angeordnet. Das Röhrchen 42 besteht vorzugsweise aus Plexiglas.

Der Katheter 16 weist weiterhin einen Mantel 44 auf, der den Lichtleiter 20 vom distalen Ende 24 bis zum proximalen Ende umgibt. Der Mantel 44 geht am distalen Ende in das distale Ende 18 des Katheters 16 über und ist dort als Vollmaterial ausgebildet. Der Mantel 44 kann aus für Katheter üblichen Materialien bestehen, wobei der Mantel 44 jedoch zumindest im Bereich der lichtablenkenden Mittel 28, das heißt im Bereich der reflektierenden Fläche 34 für das zu applizierende Licht transparent ist. Außerhalb dieses Bereiches könnte der Mantel 44 und auch das Röhrchen 42 aus einem lichtabsorbierenden Material bestehen, insbesondere schwarz sein, um Störreflexe, die nicht beteiligtes Gewebe schädigen können, zu vermeiden.

Das lichtzuführende Instrument 12 weist weiterhin Haltemittel 45 in Form eines Ballons 46 auf, der sich axial über das distale Ende 24 des Lichtleiters 20 und über die lichtablenkenden Mittel 28 hinweg erstreckt und den Lichtleiter 20 und die lichtablenkenden Mittel 28 umgibt.

Der Ballon 46 weist eine Hülle 48 auf, die wiederum zumindest im Bereich der lichtablenkenden Mittel 28 für das zu applizierende Licht transparent ist.

Der Ballon 46 ist des weiteren dilatierbar, d.h. aufweitbar, , wobei der Ballon 46 in Fig. 1 in seinem dilatierten Zustand dargestellt ist. Der zum Dilatieren des Ballons 46 erforderliche Fluideinlaß und zum Ablassen des Fluids erforderliche Fluidauslaß sind in der schematischen Darstellung nicht dargestellt. Im fluidleeren Zustand des Ballons 46 fällt dieser in sich zusammen, oder, wenn die Hülle 48 entsprechend elastisch ausgebildet ist, zieht sich entsprechend zusammen, so daß der Ballon 46 dann im Durchmesser stark verkleinert ist. Im dilatierten Zustand weist der Ballon 46 eine längsgestreckte gerade zylindrische Form auf, die sich zum Fixieren zweier Gefäße Ende an Ende aneinander eignet.

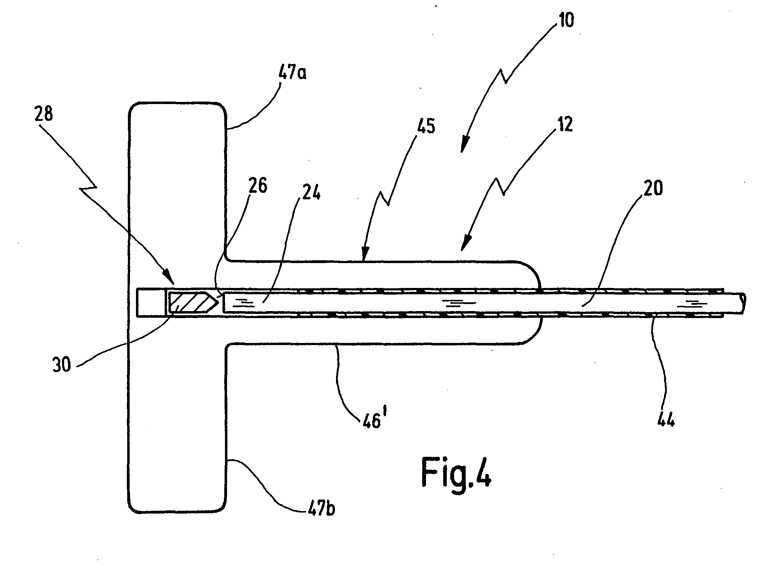

In Fig. 4 ist ein gegenüber Fig. 1 abgewandeltes Ausführungsbeispiel dargestellt, das sich zum Fixieren zweier Gefäße Ende an Seite eignet. In Fig. 4 wurden mit Fig. 1 gleiche oder entsprechende Teile mit gleichen Bezugszeichen versehen. Die Haltemittel 45 des Instruments 12 in Fig. 4 weisen einen dilatierbaren Ballon 46' auf, der T-förmig ausgebildet ist. Zwei Arme 47a und 47b des Ballons 46' verlaufen quer zur Längsrichtung des Lichtleiters 20, um bei einer Verbindung zweier Gefäße Ende an Seite in das quer zum ersten Gefäß verlaufende zweite Gefäß einzugreifen, um dieses Gefäß an dem anderen Gefäß während des Verschweißens zu fixieren.

Wieder mit Bezug auf Fig. 1 und 2 ist eine Außenseite der Hülle 48 des Ballons 46 aus einem nicht an biologischem Gewebe anhaftenden Material gefertigt.

Das lichtzuführende Instrument 12 ist insgesamt flexibel, so daß es sich an den Verlauf des Gefäßes, in das das Instrument 12 eingeführt wird, anpassen kann.

Die Lichtquelle 14 erzeugt vorzugsweise Licht in einem Wellenlängenbereich zwischen 780 nm und 2.100 nm.

Zur Erzeugung von Licht mit der Wellenlänge von 808 nm kann ein Halbleiterdiodenlaser und zur Erzeugung von Licht mit einer Wellenlänge von 2.010 nm ein diodengepumpter Ho:YAG-Laser verwendet werden.

Nachfolgend wird ein Verfahren zur Laserschweißung von zwei Gefäßstümpfen aneinander beschrieben, bei dem die Vorrichtung 10 verwendet wird. Es versteht sich jedoch, daß die Vorrichtung 10 auch für andere Behandlungen von Gefäßen mit Licht Verwendung finden kann.

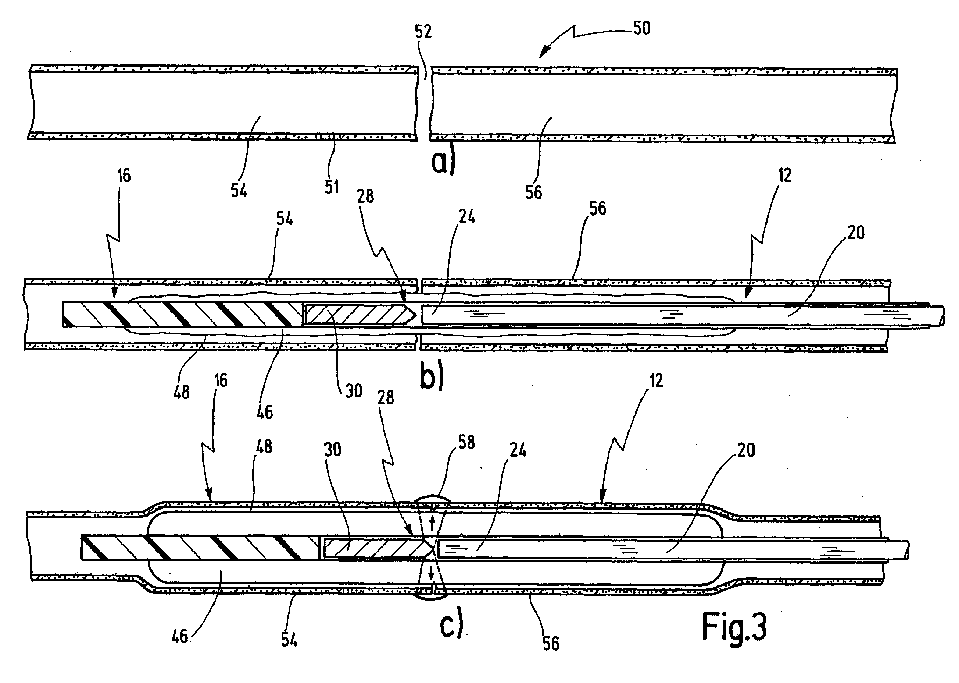

In Fig. 3a) ist schematisch ein Gefäß 50 mit einer Wand 51 dargestellt, das entlang einer Trennlinie 52 vollständig durchtrennt ist. Mit dem nachfolgend beschriebenen Verfahren sollen die entsprechenden Gefäße bzw. Gefäßstümpfe 54 und 56 an der Trennlinie 52 miteinander mittels Laserlicht verschweißt werden.

Allgemein besteht das Verfahren darin, das zum Schweißen benötigte Laserlicht durch das Lumen des Gefäßes 50 längs desselben bis zu der zu behandelnden Stelle zu führen, die hier durch die Trennlinie 52 gebildet wird, und dort das axial zugeführte Licht in radialer Richtung auf die zu behandelnde Stelle zu richten.

Vor der Lichtapplikation werden die Gefäßstümpfe 54 und 56 aneinander fixiert.

Zur Durchführung des Verfahrens ist die zuvor beschriebene Vorrichtung 10 mit dem Instrument 12 geeignet.

Das als Katheter 16 ausgebildete Instrument 12 wird zunächst in das Lumen des Gefäßes mit seinem distalen Ende 18 voran an einer von der zu behandelnden Stelle (Trennlinie 52) beabstandeten Stelle eingeführt. Die Einführung des Katheters 16 erfolgt vorzugsweise durch eine kleine Inzision, vorzugsweise dort, wo das zu behandelnde Gefäß 50 nahe der Körperoberfläche verläuft. Im Fall, daß es sich bei dem Gefäß 50 um die Baucharterie handelt, kann der Katheter 16 bspw. im Bereich der Hüftarterie, die nahe der Körperoberfläche verläuft, eingeführt werden. Der Katheter 16 weist dazu eine entsprechende Länge auf, um von der Inzisionsstelle durch das Lumen des Gefäßes 50 hindurch in dessen Längsrichtung bis zu der zu behandelnden Stelle (Trennlinie 52) vorangeschoben zu werden.

Das Instrument 12 wird im Lumen des Gefäßes 50 so weit vorgeschoben, bis die lichtablenkenden Mittel 28 auf Höhe der zu behandelnden Stelle, das heißt hier der Trennlinie 52, zu liegen kommen, wie in Fig. 3b) dargestellt ist.

Der Ballon 46 ist beim Voranschieben des Instruments 12 entleert, wie ebenfalls in Fig. 3b) dargestellt ist.

Sobald das Instrument 12 wie in Fig. 3b) an Ort und Stelle gebracht ist, erstreckt sich der Ballon 46 sowohl im Gefäßstumpf 54 als auch im Gefäßstumpf 56 über eine ausreichende Teillänge derselben.

Sobald das Instrument 12, wie in Fig. 3b) dargestellt ist, an Ort und Stelle gebracht ist, wird der Ballon 46 durch am Instrument 12 vorgesehene geeignete Einrichtungen, die von extrakorporal bedienbar sind, dilatiert, so daß der Ballon 46 den in Fig. 3c) dargestellten Zustand einnimmt.

Der Ballon 46 läßt sich vorzugsweise so weit dilatieren, daß sein Außendurchmesser geringfügig größer ist als der Innendurchmesser des Lumens des Gefäßes 50, so daß das Gefäß 50 im Bereich des Ballons 46 etwas radial gedehnt wird. Auf diese Weise sind die Gefäßstümpfe 54 und 56 auf dem Ballon 46 und somit an dem Instrument 12 unbeweglich fixiert und ohne Achsenversatz exakt zueinander ausgerichtet.

Die Gefäßstümpfe 54 und 56 werden entlang der Trennlinie 52 möglichst nahe aneinander zusammengeschoben.

Nunmehr kann mit dem Laserschweißen begonnen werden. Dazu wird die Lichtquelle 14 eingeschaltet und das von der Lichtquelle 14 erzeugte Licht wird entlang dem Lichtleiter 20 in die Nähe der zu behandelnden Stelle in der Wand des Gefäßes 50, das heißt in Nähe der Trennlinie 52 geführt, tritt dort aus dem distalen Ende 24 des Lichtleiters 20 aus und wird über die lichtablenkenden Mittel 28 im wesentlichen radial, und zwar über den vollen Umfang der Trennlinie 52 gleichmäßig verteilt auf das Gewebe der Wand des Gefäßes 50 in einem kleinen Bereich beidseits der Trennlinie 52 gelenkt. Das Licht tritt dabei durch den Ballon 46 hindurch in das Gewebe der Wand des Gefäßes 50 ein.

Wenn ohne ein biologisches Lot gearbeitet wird, wird Laserlicht mit einer Wellenlänge von 2010 nm verwendet. Der Spotdurchmesser des auf das Gewebe des Gefäßes 50 auftreffenden Lichts hat in axialer Richtung des Gefäßes 50 eine Abmessung von etwa 4 mm. Die Einstrahldauer beträgt zwischen etwa 50 s und 140 s, bis das Gewebe der Gefäßstümpfe 54 und 56 miteinander verschmolzen ist.

Bei Verwendung von Laserlicht einer Wellenlänge von 808 nm wird im Bereich der Trennlinie 52 von der Außenseite des Gefäßes 50 her zuvor bspw. mit einer Spritze ein biologisches Lot 58, bspw. Albumin, vor dem Bestrahlen mit Licht aufgebracht. Das Lot ist geeignet, durch Lichtabsorption mit dem Gewebe des Gefäßes 50 zu koagulieren und somit den gewünschten Bindungseffekt zu verstärken. Das Laserlicht tritt bei dieser Wellenlänge im wesentlichen absorptionsfrei durch die Gefäßwand hindurch und wird vollständig von dem Lot 58 absorbiert.

Aus der vorstehenden Beschreibung geht hervor, daß mittels der erfindungsgemäßen Vorrichtung 10 und dem erfindungsgemäßen Instrument 12 das Verfahren zum Laserschweißen zweier Gefäße aneinander durch die aus dem Lumen des Gefäßes her erfolgende gleichmäßig radial nach allen Seiten hin erfolgende Lichteinstrahlung zum einen eine Verkürzung der Behandlungsdauer und auch eine allseitig gleichmäßige Einwirkung des Lichts ermöglicht.

## Patentansprüche

1. Vorrichtung zum Applizieren von Licht auf eine zu behandelnde Stelle einer Wand (51) eines Gefäßes (50) im menschlichen oder tierischen Körper, insbesondere zum Laserschweißen zweier Gefäße aneinander, mit einem lichtzuführenden Instrument (12), das von einer extrakorporalen Lichtquelle (14) erzeugtes Licht zu der Stelle führt und auf diese abstrahlt, wobei das lichtzuführende Instrument (12) einen länglichen Lichtleiter (20), der in das Lumen des Gefäßes (50) einführbar und darin in Längsrichtung des Gefäßes (50) verschiebbar ist, wobei das lichtzuführende Instrument (12) lichtablenkende Mittel (28) aufweist, die das durch den Lichtleiter (20) zugeführte Licht im wesentlichen radial auf die zu behandelnde Stelle richten, wobei die lichtablenkenden Mittel (28) so ausgebildet sind, daß sie das aus dem Lichtleiter (20) austretende Licht ohne Drehen des Lichtleiters (20) um seine Längsachse über einen vollen Umfang gleichmäßig verteilt ringförmig zur zu behandelnden Stelle an der Wand (51) des Gefäßes (50) hin lenken, wobei die lichtablenkenden Mittel (28) als Reflektor (30) ausgebildet sind und der Reflektor (30) eine reflektierende Fläche (34) aufweist, die sich vollumfänglich um die Längsrichtung des Lichtleiters (20) erstreckt, **dadurch gekennzeichnet, dass** die reflektierende Fläche (34) zur Längsrichtung des Lichtleiters (20) unter einem Winkel im Bereich zwischen 40° und 50° geneigt ist.

2. Vorrichtung nach Anspruch 1, **dadurch gekennzeichnet, daß** die reflektierende Fläche (34) zur Längsrichtung des Lichtleiters (20) unter einem Winkel von etwa 45° geneigt ist.

3. Vorrichtung nach Anspruch 1 oder 2, **dadurch gekennzeichnet, daß** die reflektierende Fläche (34) als Konusfläche, insbesondere spitzkegelförmig ausgebildet ist.

4. Vorrichtung nach einem der Ansprüche 1 bis 3, **dadurch gekennzeichnet, daß** die reflektierende Fläche (34) gerade, konkav gekrümmt oder konvex gekrümmt ist.

5. Vorrichtung nach einem der Ansprüche 1 bis 4, **dadurch gekennzeichnet, daß** das distale Ende (24) des Lichtleiters (20) und die lichtablenkenden Mittel (28) relativ zueinander unbeweglich gehalten sind.

6. Vorrichtung nach einem der Ansprüche 1 bis 5, **dadurch gekennzeichnet, daß** das lichtzuführende Instrument (12) als Katheter (16) ausgebildet ist.

7. Vorrichtung nach Anspruch 6, **dadurch gekennzeichnet, daß** der Katheter (16) einen den Lichtleiter umhüllenden Mantel (44) aufweist, der zumindest im Bereich der lichtablenkenden Mittel (28) für das zu applizierende Licht transparent ist.

8. Vorrichtung nach Anspruch 6 oder 7, **dadurch gekennzeichnet, daß** das distale Ende (24) des Lichtleiters (20) und die lichtablenkenden Mittel (28) in einem beide verbindenden, für das zu applizierende Licht transparenten Röhrchen (42) angeordnet sind.

9. Vorrichtung nach einem der Ansprüche 1 bis 8, **dadurch gekennzeichnet, daß** das lichtzuführende Instrument (12) Haltemittel (45) zum Fixieren des Gefäßes (50), insbesondere von Gefäßen aneinander, während der Lichtapplikation aufweist.

10. Vorrichtung nach Anspruch 9, **dadurch gekennzeichnet, daß** die Haltemittel (45) einen Ballon (46) aufweisen, der sich axial über das distale Ende (24) des Lichtleiters (20) und über die lichtablenkenden Mittel (28) hinweg erstreckt und den Lichtleiter (20) und die lichtablenkenden Mittel (28) umgibt.

11. Vorrichtung nach Anspruch 10, **dadurch gekennzeichnet, daß** der Ballon (46) eine langerstreckte gerade oder T-förmige Geometrie aufweist.

12. Vorrichtung nach Anspruch 10 oder 11, **dadurch gekennzeichnet, daß** der Ballon (46) dilatierbar ist.

13. Vorrichtung nach einem der Ansprüche 10 bis 12, **dadurch gekennzeichnet, daß** eine Außenseite des Ballons (46) aus einem nicht an biologischem Gewebe anhaftenden Material besteht.

14. Vorrichtung nach einem der Ansprüche 10 bis 13, **dadurch gekennzeichnet, daß** der Ballon (46) zumindest im Bereich der lichtablenkenden Mittel (28) für das zu applizierende Licht transparent ist.

15. Vorrichtung nach einem der Ansprüche 1 bis 14, **dadurch gekennzeichnet, daß** das lichtzuführende Instrument (20) flexibel ist.

16. Vorrichtung nach einem der Ansprüche 1 bis 15, **dadurch gekennzeichnet, daß** der Lichtleiter (20) eine einzelne Lichtleitfaser aufweist.

17. Vorrichtung nach einem der Ansprüche 1 bis 16, **dadurch gekennzeichnet, daß** die Lichtquelle (14) Licht erzeugt, dessen Wellenlänge in einem wellenlängenbereich zwischen 780 nm und 2100 nm liegt und vorzugsweise 808 nm oder 2010 nm beträgt.

## Claims

1. An apparatus for applying light to a site to be treated on a wall (51) of a vessel (50) in the human or animal body, in particular for laser welding of two vessels to one another, comprising a light-feeding instrument (12) that guides light generated by an extracorporeal light source (14) to the site and radiates it onto the latter, wherein the light-feeding instrument (12) has an elongated light guide (20) that can be inserted into the lumen of the vessel (50) and can be displaced therein in the longitudinal direction of the vessel (50), wherein the light-feeding instrument (12) has light-deflecting means (28) that direct the light fed through the light guide (20) in a substantially radial fashion onto the site to be treated, wherein the light-deflecting means (28) are designed such that they direct the light emerging from the light guide (20) toward the site to be treated on the wall (51) of the vessel (50) in a fashion distributed uniformly over its entire circumference and in annular fashion without rotation of the light guide (20) about its longitudinal axis, wherein the light-deflecting means (28) are designed as a reflector (30) and the reflector (30) has a reflecting surface (34), which extends over the full circumference about the longitudinal direction of the light guide (20), **characterized in that** the reflecting surface (34) is inclined to the longitudinal direction of the light guide (20) at an angle in the range between 40° and 50°.

2. The apparatus of claim 1, **characterized in that** the reflecting surface (34) is inclined to the longitudinal direction of the light guide (20) at an angle of approximately 45°.

3. The apparatus of claim 1 or 2, **characterized in that** the reflecting surface (34) is designed as a conical surface, in particular in the shape of a tipped cone.

4. The apparatus of anyone of claims 1 through 3, **characterized in that** the reflecting surface (34) is straight, concavely curved or convexly curved.

5. The apparatus of anyone of claims 1 through 4, **characterized in that** the distal end (24) of the light guide (20) and the light-deflecting means (28) are held immovably relative to one another.

6. The apparatus of anyone of claims 1 through 5, **characterized in that** the light-feeding instrument (12) is designed as a catheter (16).

7. The apparatus of claim 6, **characterized in that** the catheter (16) has a jacket (44) that encases the light guide and is transparent to the light to be applied, at least in the region of the light-deflecting means (28).

8. The apparatus of claim 6 or 7, **characterized in that** the distal end (24) of the light guide (20) and the light-deflecting means (28) are arranged in a tube (42) that connects the two and is transparent to the light to be applied.

9. The apparatus of anyone of claims 1 through 8, **characterized in that** the light-feeding instrument (12) has holding means (45) for fixing the vessel (50), in particular for fixing vessels to one another, during the application of light.

10. The apparatus of claim 9, **characterized in that** the holding means (45) have a balloon (46) that extends axially beyond the distal end (24) of the light guide (20) and beyond the light-deflecting means (28) and surrounds the light guide (20) and the light-deflecting means (28).

11. The apparatus of claim 10, **characterized in that** the balloon (46) has an elongated straight or T-shaped geometry.

12. The apparatus of claim 10 or 11, **characterized in that** the balloon (46) can be dilated.

13. The apparatus of anyone of claims 10 through 12, **characterized in that** an outer side of the balloon (46) consists of a material that does not adhere to biological tissue.

14. The apparatus of anyone of claims 10 through 13, **characterized in that** the balloon (46) is transparent to the light to be applied at least in the region of the light-deflecting means (28).

15. The apparatus of anyone of claims 1 through 14, **characterized in that** the light-feeding instrument (20) is flexible.

16. The apparatus of anyone of claims 1 through 15, **characterized in that** the light guide (20) has a single optical fiber.

17. The apparatus of anyone of claims 1 through 16, **characterized in that** the light source (14) generates light whose wavelength is in a wavelength region between 780 nm and 2100 nm, and preferably is 808 nm or 2010 nm.

## Revendications

1. Dispositif destiné à appliquer une lumière sur une zone à traiter d'une paroi (51) d'un vaisseau (50) dans le corps humain ou animal, en particulier pour le soudage par laser de deux vaisseaux l'un à l'autre, comportant un instrument (12) pour l'acheminement de la lumière, qui guide la lumière générée par une source de lumière (14) extracorporelle vers la zone à traiter et la projette sur ladite zone, ledit instrument (12) pour l'acheminement de la lumière comportant un conducteur optique (20) allongé, qui peut être introduit dans l'orifice du vaisseau (50) et peut être déplacé dans ce dernier dans la direction longitudinale du vaisseau (50), ledit instrument (12) pour l'acheminement de la lumière comportant des moyens de déviation de la lumière (28), par lesquels la lumière acheminée par le conducteur optique (20) est dirigée sensiblement radialement sur la zone à traiter, les moyens de déviation de la lumière (28) étant réalisés de telle sorte que, sans la rotation du conducteur optique (20) autour de son axe longitudinal, ils guident vers la zone à traiter sur la paroi (51) du vaisseau (50) la lumière sortant du conducteur optique (20) en la projetant en forme d'anneau de manière homogène sur tout le pourtour, lesdits moyens de déviation de la lumière (28) étant réalisés sous forme de réflecteur (30) et ledit réflecteur (30) comportant une surface (34) réfléchissante, qui s'étend sur tout le pourtour autour de la direction longitudinale du conducteur optique (20), **caractérisé en ce que** la surface (34) réfléchissante est inclinée par rapport à la direction longitudinale du conducteur optique (20) en formant un angle dans une plage entre 40° et 50°.

2. Dispositif selon la revendication 1, **caractérisé en ce que** la surface (34) réfléchissante est inclinée par rapport à la direction longitudinale du conducteur optique (20) en formant un angle de 45° environ.

3. Dispositif selon la revendication 1 ou 2, **caractérisé en ce que** la surface (34) réfléchissante est réalisée sous forme de surface conique, en particulier sous forme de cône pointu.

4. Dispositif selon l'une quelconque des revendications 1 à 3, **caractérisé en ce que** la surface (34) réfléchissante est droite, courbée en concave ou courbée en convexe.

5. Dispositif selon l'une quelconque des revendications 1 à 4, **caractérisé en ce que** l'extrémité distale (24) du conducteur optique (20) et les moyens de déviation de la lumière (28) sont maintenus de manière immobile les uns par rapport aux autres.

6. Dispositif selon l'une quelconque des revendications 1 à 5, **caractérisé en ce que** l'instrument (12) pour l'acheminement de la lumière est réalisé sous forme de cathéter (16).

7. Dispositif selon la revendication 6, **caractérisé en ce que** le cathéter (16) comporte une paroi latérale (44), qui enveloppe le conducteur optique et qui, au moins dans la zone des moyens de déviation de la lumière (28), est transparente à la lumière à appliquer.

8. Dispositif selon la revendication 6 ou 7, **caractérisé en ce que** l'extrémité distale (24) du conducteur optique (20) et les moyens de déviation de la lumière (28) sont disposés dans un petit tube (42), les reliant et transparent à la lumière à appliquer.

9. Dispositif selon l'une quelconque des revendications 1 à 8, **caractérisé en ce que** l'instrument (12) pour l'acheminement de la lumière comporte des moyens de fixation (45) pour immobiliser le vaisseau (50), en particulier les vaisseaux l'un à l'autre, pendant l'application de la lumière.

10. Dispositif selon la revendication 9, **caractérisé en ce que** les moyens de fixation (45) comportent un ballonnet (46), qui s'étend axialement au-delà de l'extrémité distale (24) du conducteur optique (20) et au-delà des moyens de déviation de la lumière (28) et qui entoure le conducteur optique (20) et les moyens de déviation de la lumière (28).

11. Dispositif selon la revendication 10, **caractérisé en ce que** le ballonnet (46) possède une forme géométrique allongée droite ou en forme de T.

12. Dispositif selon la revendication 10 ou 11, **caractérisé en ce que** le ballonnet (46) peut se dilater.

13. Dispositif selon l'une quelconque des revendications 10 à 12, **caractérisé en ce qu'**une face extérieure du ballonnet (46) est réalisée dans un matériau n'adhérant pas au tissu biologique.

14. Dispositif selon l'une quelconque des revendications 10 à 13, **caractérisé en ce que** le ballonnet (46), au moins dans la zone des moyens de déviation de la lumière (28), est transparent à la lumière à appliquer.

15. Dispositif selon l'une quelconque des revendications 1 à 14, **caractérisé en ce que** l'instrument (20) pour l'acheminement de la lumière est flexible.

16. Dispositif selon l'une quelconque des revendications 1 à 15, **caractérisé en ce que** le conducteur optique (20) comporte une seule fibre optique.

17. Dispositif selon l'une quelconque des revendications 1 à 16, **caractérisé en ce que** la source de lumière (14) génère une lumière dont la longueur d'ondes se situe dans une plage entre 780 nm et 2 100 nm et de préférence une longueur d'ondes de 808 nm ou de 2 010 nm.
